# EUROPEAN PATENT APPLICATION

(11) **EP 4 400 578 A1**
(43) Date of publication of application: **17.07.2024**
(21) Application number: 22867393.5
(22) Date of filing: 08.09.2022
(51) Int. Cl.: C12N 1/20, C12Q 1/02, A23C 9/123

(54) **LACTIC ACID BACTERIUM, LACTIC ACID BACTERIUM STARTER, FERMENTED MILK, METHOD FOR MANUFACTURING FERMENTED MILK, AND METHOD FOR SCREENING LACTIC ACID BACTERIUM**

(30) Priority: 09.09.2021 JP 2021147151
(71) Applicant: MEIJI CO., LTD, Chuo-ku Tokyo 104-8306 (JP)
(72) Inventor: GOTO Hirofumi, Hachioji-shi, Tokyo 192-0919 (JP); MIZOGUCHI Chinami, Hachioji-shi, Tokyo 192-0919 (JP); MAKABE Yoshimi, Hachioji-shi, Tokyo 192-0919 (JP)
(74) Representative: TBK
(86) International application number: PCT/JP2022/033646
(87) International publication number: WO 2023/038072

(57) **Abstract**

A lactic acid bacterium belonging to Streptococcus thermophilus, satisfying at least one of the conditions selected from the group consisting of the following (a) and (b):
(a) when cultured at 43°C in a 10% skimmed milk powder medium, (a1) the time from start of the culture until a pH of a culture solution becomes 4.7 or lower is 7 hours or less, and (a2) the pH of the culture solution 20 hours after the start of the culture becomes 4.1 or higher, and
(b) when cultured until the pH of the culture solution becomes 4.7 or less in a 10% skimmed milk powder medium at 43°C, followed by storage at 10°C for 10 days, (b1) a viable bacterial count in the culture solution after the storage becomes 1 × 10⁸ cfu/g or more, and (b2) the pH of the culture solution after the storage becomes 4.2 or higher.

## Description

### [Technical Field]

The present invention relates to lactic acid bacterium, a lactic acid bacterium starter, fermented milk, and a method for producing fermented milk, and more particularly to lactic acid bacterium, a lactic acid bacterium starter and fermented milk comprising the same, and a method for producing fermented milk, and further a method for screening lactic acid bacterium.

### [Background Art]

For example, in Japan's "Ministerial Ordinance on Milk and Milk Products Concerning Compositional Standards, etc. (Ministerial Ordinance on Milk and Milk Products)", fermented milk is defined as "products which are obtained by fermenting milk, or milk, etc. comprising an equal or greater amount of milk solids-not-fat with lactic acid bacteria or yeast and then forming a paste or liquid, or the frozen product." Representative examples of such fermented milk include yogurt such as set type yogurt (solid fermented milk), soft type yogurt (pasty fermented milk), and drink type yogurt (liquid fermented milk). Note that, for example, in the "Codex Alimentarius (FAO (Food and Agriculture Organization of the United Nations)/WHO (World Health Organization))", which is a food standard shared by the international community, yogurt is defined as "any food that is made through the process of lactic acid fermentation combining Lactobacillus delbrueckii subsp. bulgaricus and Streptococcus thermophilus (S. thermophilus)." Traditionally, yogurt is generally characterized by adding these two types of bacteria as starters to raw material milk and fermenting lactose in the raw material milk, primarily characterized by its sourness due to the lactic acid produced during fermentation.

However, lactic acid bacteria such as Streptococcus thermophilus are capable of producing lactic acid through fermentation even at low temperatures. Therefore, in the fermented products after fermentation (for example, fermented milk such as yogurt), during storage, even under low-temperature conditions, an excessive production of lactic acid leads to an increase in acidity (that is, decrease in pH), posing a problem of compromising its flavor.

Consequently, research efforts have been undertaken to inhibit the acid production in such lactic acid bacteria at low temperatures. For instance, Japanese Patent Application Publication No. Hei 7-236416 (PTL 1) describes the use of acid production inhibition strains of Lactobacillus delbrueckii subsp. bulgaricus and viscosity producing strains of Streptococcus salivarius subsp. thermophilus to obtain fermented milk with minimal increase in acidity during low-temperature storage. Furthermore, Japanese Patent No. 4331309 (PTL 2) has been granted for a lactic acid bacterial strain belonging to the low-temperature-sensitive Streptococcus thermophilus, which coagulates 12% reduced skim milk with 0.05% yeast extract addition within 3 hours at 37 to 43°C but does not coagulate 12% reduced skim milk with 0.05% yeast extract addition within 3 days at 15 to 25°C. Specifically, Streptococcus thermophilus SBT0144 is described as one of the lactic acid bacterial strains. Moreover, Japanese Patent Application Publication No. 2001-95561 (PTL 3) describes mutant strains of Lactobacillus delbrueckii subsp. bulgaricus with reduced cell membrane-bound adenosine triphosphatase (ATPase) activity. In these mutant strains, a decrease in pH during the fermentation process leads to intracellular pH reduction due to the retention of H⁺ within the cells, inhibiting growth (that is, inducing so-called apoptosis). As a result, the production of lactic acid decreases.

### [Citation List]

### [Patent Literature]

[PTL 1] Japanese Patent Application Publication No. Hei 7-236416
[PTL 2] Japanese Patent No. 4331309
[PTL 3] Japanese Patent Application Publication No. 2001-95561

### [Summary of Invention]

### [Technical Problem]

The flavor of fermented milk (such as sourness, milkiness, and sweetness) mainly depends on the characteristics of various lactic acid bacteria. Therefore, in fermented milk that requires combining specific bacterial strains as described in PTL 1, there is a problem of obtaining only limited-flavor fermented milk. Additionally, through research on lactic acid bacteria for fermented milk and the production thereof, the present inventors have found that when using Streptococcus thermophilus SBT0144 (hereinafter referred to as "SBT0144 strain" as needed) as described in PTL 2, although the acid production at low temperatures decreases and the pH reduction is indeed suppressed in the obtained fermented milk, there are problems that it takes time for fermentation to complete (for example, until the pH reaches 4.7 or below), and viable bacterial count of lactic acid bacteria decreases during low-temperature storage, similar to the mutant strain described in PTL 3.

The present invention has been made in view of the above problems posed by the related art, and aims to provide lactic acid bacterium which allows for obtaining fermented milk in which the decrease in pH during low-temperature storage is suppressed, and the viable bacterial count of the lactic acid bacteria is sufficiently maintained even by the low-temperature storage, a lactic acid bacterium starter and fermented milk comprising the same, and a method for producing fermented milk, and further a method for screening lactic acid bacterium.

### [Solution to Problem]

The present inventors have diligently conducted research in order to achieve the above objects, and as a result, under specific conditions, namely, (a) when cultured at 43°C in a 10% skimmed milk powder medium, (a1) the time from start of the culture until a pH of a culture solution becomes 4.7 or lower is 7 hours or less, and (a2) the pH of the culture solution 20 hours after the start of the culture becomes 4.1 or higher, and/or (b) when cultured until the pH of the culture solution becomes 4.7 or less in a 10% skimmed milk powder medium at 43°C, followed by storage at 10°C for 10 days, (b1) a viable bacterial count in the culture solution after the storage becomes 1 × 10⁸ cfu/g or more, and (b2) the pH of the culture solution after the storage becomes 4.2 or higher, in the fermented milk obtained by the lactic acid bacteria that fulfill these conditions, the decrease in pH during low-temperature storage is suppressed, and the viable bacterial count is sufficiently maintained even by the low-temperature storage. Thus, the present invention has been completed.

The aspects of the present invention obtained from such findings are as follows.
[1] A lactic acid bacterium belonging to Streptococcus thermophilus, satisfying at least one of the conditions selected from the group consisting of the following (a) and (b):
   (a) when cultured at 43°C in a 10% skimmed milk powder medium, (a1) the time from start of the culture until a pH of a culture solution becomes 4.7 or lower is 7 hours or less, and (a2) the pH of the culture solution 20 hours after the start of the culture becomes 4.1 or higher, and
   (b) when cultured until the pH of the culture solution becomes 4.7 or less in a 10% skimmed milk powder medium at 43°C, followed by storage at 10°C for 10 days, (b1) a viable bacterial count in the culture solution after the storage becomes 1 × 10⁸ cfu/g or more, and (b2) the pH of the culture solution after the storage becomes 4.2 or higher.
[2] The lactic acid bacterium according to [1], satisfying the condition (a) and further satisfying the following (a3) or (a4):
   (a3) in the culture, after the culture until the pH of the culture solution becomes 4.7 or lower, the viable bacterial count in the culture solution after 10 days of storage at 10°C becomes 1 × 10⁸ cfu/g or higher, or
   (a4) in the culture, after the culture until the pH of the culture solution becomes 4.7 or lower, the pH in the culture solution after 10 days of storage at 10°C becomes 4.2 or higher.
[3] The lactic acid bacterium according to [1], satisfying the condition (b) and further satisfying the following (b3) or (b4):
   (b3) the time until the pH of the culture solution becomes 4.7 or lower is 7 hours or less from the start of the culture, or
   (b4) the pH of the culture solution after 20 hours from the start of the culture is 4.1 or higher.
[4] The lactic acid bacterium according to any one of [1] to [3], possessing a prtS gene.
[5] The lactic acid bacterium according to any one of [1] to [4], which is at least one selected from the group consisting of Streptococcus thermophilus OLS4801 (accession number: NITE BP-03504), Streptococcus thermophilus OLS4802 (accession number: NITE BP-03505), Streptococcus thermophilus OLS4803 (accession number: NITE BP-03506), Streptococcus thermophilus OLS4823 (accession number: NITE BP-03507), and Streptococcus thermophilus OLS4824 (accession number: NITE BP-03508).
[6] A lactic acid bacterium composition comprising: the lactic acid bacterium according to any one of [1] to [5].
[7] The lactic acid bacterium composition according to [6], further comprising: a lactic acid bacterium of the genus Lactobacillus.
[8] The lactic acid bacterium composition according to [6] or [7], which is a lactic acid bacterium starter.
[9] The lactic acid bacterium composition according to [6] or [7], which is fermented milk.
[10] A method for producing fermented milk comprising: a fermentation step of adding the lactic acid bacterium according to any one of [1] to [5] or the lactic acid bacterium composition according to any one of [6] to [9] to a formula milk comprising raw material milk, followed by fermentation to thereby obtain fermented milk.
[11] The method for producing fermented milk according to [10], further comprising: adding a lactic acid bacterium of the genus Lactobacillus to the formula milk.
[12] A method for screening lactic acid bacterium, comprising: a selection step of selecting lactic acid bacterium with low acid production using an indicator that a lactic acid bacterium belonging to Streptococcus thermophilus satisfies the following condition (a):
   (a) when cultured at 43°C in a 10% skimmed milk powder medium, (a1) the time from start of the culture until a pH of a culture solution becomes 4.7 or lower is 7 hours or less, and (a2) the pH of the culture solution 20 hours after the start of the culture becomes 4.1 or higher.
[13] A method for producing fermented milk comprising: a fermentation step of adding lactic acid bacterium selected by the method for screening lactic acid bacterium according to [12] to a formula milk comprising raw material milk, followed by fermentation to thereby obtain fermented milk.

### [Advantageous Effects of Invention]

The present invention makes it possible to provide lactic acid bacterium which allows for obtaining fermented milk in which the decrease in pH during low-temperature (such as 10°C) storage is suppressed, and the viable bacterial count of the lactic acid bacteria is sufficiently maintained even by the low-temperature storage, a lactic acid bacterium starter and fermented milk comprising the same, and a method for producing fermented milk, and further a method for screening lactic acid bacterium.

### [Brief Description of Drawings]

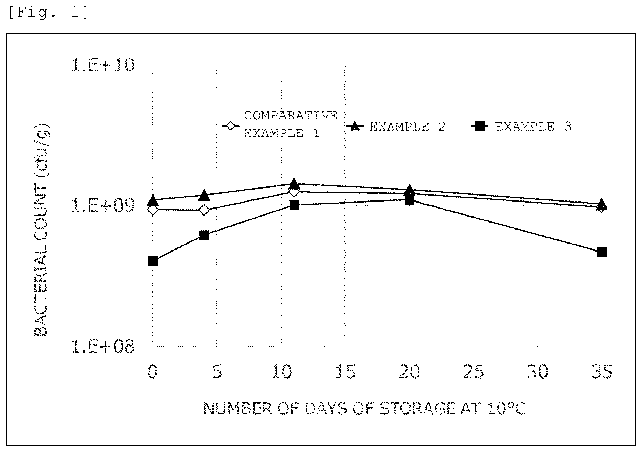

[Fig. 1] Fig. 1 is a graph illustrating the time course of viable bacterial counts during storage at 10°C for OLS4802 strain (Example 2), OLS4803 strain (Example 3), and P2101201 strain (Comparative Example 1) in Test Example 3.

### [Description of Embodiments]

Hereinafter, the present invention will be described in detail with reference to preferred embodiments thereof.

### <Lactic Acid Bacteria>

In the present invention, the term "lactic acid bacterium (bacteria)" refers to a collective term for microorganisms capable of assimilating glucose and producing lactic acid at a yield of 50% or more based on sugar, and as physiological characteristics, they are gram-positive cocci or rods, non-motile, often lacking the ability to form spores (although some lactic acid bacteria, such as Bacillus coagulans, have the ability to form spores), and possess features such as being catalase-negative. Among others, the lactic acid bacterium of the present invention belong to Streptococcus thermophilus.

### (Streptococcus thermophilus)

Streptococcus thermophilus, also referred to as thermophilus bacterium, is a streptococcus capable of producing lactic acid from lactose, thus being a lactic acid bacterium. In the present specification, lactic acid bacterium (bacteria) belonging to Streptococcus thermophilus are sometimes referred to as "S. thermophilus."

The lactic acid bacterium of the present invention, as S. thermophilus, preferably possess the prtS gene. By employing S. thermophilus that possesses the prtS gene, it is possible to shorten the fermentation time until completion and further trend towards obtaining fermented milk with a more preferable flavor. In the present invention, the term "prtS gene" refers to a gene encoding a cell-wall-bound serine protease that degrades casein. Additionally, in the present invention, the possession of the prtS gene by S. thermophilus can be determined, for example, by selecting a highly conserved sequence among the prtS genes of S. thermophilus obtained from public databases (such as GenBank), amplifying a portion of the prtS gene by PCR using a primer set prepared based on the selected sequence, and confirming the desired PCR product. As an example of such a primer set, the primer set composed of Fprimer and Rprimer described in the following examples can be mentioned, but this is not limiting.

### (Conditions (a) and (b))

The lactic acid bacteria of the present invention are required to satisfy at least one of the conditions selected from the group consisting of the following (a) and (b):
(a) when cultured at 43°C in a 10% skimmed milk powder medium,
   (a1) the time from start of the culture until a pH of a culture solution becomes 4.7 or lower is 7 hours or less, and
   (a2) the pH of the culture solution 20 hours after the start of the culture becomes 4.1 or higher, and
(b) when cultured until the pH of the culture solution becomes 4.7 or less in a 10% skimmed milk powder medium at 43°C, followed by storage at 10°C for 10 days,
   (b1) a viable bacterial count in the culture solution after the storage becomes 1 × 10⁸ cfu/g or more, and
   (b2) the pH of the culture solution after the storage becomes 4.2 or higher.

By satisfying the above condition (a) and/or condition (b) with the lactic acid bacteria, in the fermented milk obtained using such lactic acid bacteria, it becomes possible to reduce the amount of acid production during low-temperature storage, suppress pH decrease, and sufficiently maintain the viable bacterial count of the lactic acid bacteria even during the low-temperature storage. In particular, the present inventors have surprisingly found a correlation between the pH changes in the culture solution during culture at 43°C and the pH changes in the culture solution during storage at 10°C, and according to the lactic acid bacteria that satisfy condition (a), the above effects of the present invention are achieved.

If the lactic acid bacteria satisfy only the conditions (a) mentioned above (namely, conditions (a1) and (a2)), in addition to these conditions (a), it is preferable to further satisfy the following (a3) and/or (a4):
(a3) in the culture, after the culture until the pH of the culture solution becomes 4.7 or lower, the viable bacterial count in the culture solution after 10 days of storage at 10°C becomes 1 × 10⁸ cfu/g or higher, and/or
(a4) in the culture, after the culture until the pH of the culture solution becomes 4.7 or lower, the pH in the culture solution after 10 days of storage at 10°C becomes 4.2 or higher.

In addition, if the lactic acid bacteria satisfy only the conditions (b) mentioned above (namely, conditions (b1) and (b2)), in addition to these conditions (b), it is preferable to further satisfy the following conditions (b3) and/or (b4):
(b3) the time until the pH of the culture solution becomes 4.7 or lower is 7 hours or less from the start of the culture, and/or
(b4) the pH of the culture solution after 20 hours from the start of the culture is 4.1 or higher.

### [Skimmed Milk Powder Medium]

In conditions (a) and (b), "10% skimmed milk powder medium" refers to a medium comprising 10% by mass of skimmed milk powder. The skimmed milk powder according to the present invention refers to defatted animal milk (preferably cow's milk), formed into skim milk and dried into a powdered form, and examples thereof include those having 95.0% or more of milk solids and 5.0% or less of moisture content, as stipulated in Japan's "Ministerial Ordinance on Milk and Milk Products Concerning Compositional Standards, etc. (hereinafter sometimes referred to as 'Ministerial Ordinance on Milk and Milk Products')." The composition of such skimmed milk powder includes, for example, the composition described in the "Standards Tables of Food Composition in Japan -2015- (Seventh Revised Edition)," that is, per 100 g, 300 to 400 (preferably 359) kcal of energy, 3 to 5 (preferably 3.8) g of moisture, 30 to 40 (preferably 34.0) g of protein, 0.2 to 2 (preferably 1.0) g of fat, 45 to 60 (preferably 53.3) g of carbohydrates (including lactose), 900 to 2000 (preferably 1100) mg of calcium, 3 to 10 (preferably 6) µg of vitamin A, 0.5 to 2 (preferably 1.60) mg of vitamin B₂, 10 to 30 (preferably 25) mg of cholesterol, and 0.5 to 2 (preferably 1.4) g of equivalent salt content. The pH of such 10% skimmed milk powder medium is typically within the range of 6 to 7.

For the 10% skimmed milk powder medium according to conditions (a) and (b), it is permissible to include additional components besides skimmed milk powder; however, even in such cases, it is preferable that the content of the additional components be 0.1% by mass or less, and more preferably 0.01% by mass or less, with the medium composed of 10% by mass of skimmed milk powder and 90% by mass of water (preferably potable water) being particularly preferable. As the aforementioned additional components, for example, yeast extract and peptides are mentioned, but especially for the 10% skimmed milk powder medium according to conditions (a) and (b), it is preferable that the yeast extract is substantially absent. Note that, in the present invention, "substantially absent" refers to being 0.001% by mass or less with respect to the total mass. The lactic acid bacteria of the present invention can fulfill condition (a) and/or condition (b) even in a medium not comprising the yeast extract.

For the 10% skimmed milk powder medium according to conditions (a) and (b), it is preferable that total sterilization treatment or partial sterilization treatment has been applied. Although the conditions therefore are not specifically limited, conditions for the total sterilization treatment can include temperatures of 70 to 130°C for 1 minute to 1 hour, and conditions for the partial sterilization treatment can include temperatures of 75 to 125°C for 10 to 30 minutes. Additionally, for the 10% skimmed milk powder medium, it may have the same composition and/or the same total sterilization treatment or partial sterilization treatment as the first 10% skimmed milk powder medium and the second 10% skimmed milk powder medium below, or it may have different compositions and/or treatments. Furthermore, when conducting the activation culture below, the second 10% skimmed milk powder medium may comprise a part or all of the culture solution after the first culture.

### [Activation Culture (First Culture)]

In conditions (a) and (b), when lactic acid bacteria have been stored at low temperatures or frozen, it is preferable to first perform activation culture on the lactic acid bacteria. As such activation culture (hereinafter sometimes referred to as "first culture"), a 10% skimmed milk powder medium (hereinafter, the medium for the first culture is sometimes referred to as "first 10% skimmed milk powder medium") is used, and culture is preferably conducted at 37°C, anaerobically, for 16 to 18 hours (preferably 16 hours). In this case, the amount of lactic acid bacteria subjected to the first culture is preferably an amount that results in a viable bacterial count of 1 × 10² to 2 × 10⁸ cfu/g, and more preferably 1 × 10⁴ to 1 × 10⁶ cfu/g, in the first 10% skimmed milk powder medium.

Furthermore, in the present invention, measurement of viable bacterial count can be carried out, for example, by culturing a diluted solution of lactic acid bacteria-comprising liquid (for example, in the case of the first culture, the culture solution of the first culture (lactic acid bacteria and first 10% skimmed milk powder medium)), suitably diluted, on a suitable agar medium, such as BCP added plate count agar medium defined by the Ministerial Ordinance on Milk and Milk Products, and counting the number of colonies that appear. The value of viable bacterial count (cfu/g) in this case is the viable bacterial count in the lactic acid bacteria-comprising liquid (g), which hence can be used to calculate the viable bacterial count in the culture solution of the first culture, for instance.

As for the anaerobic culture method, although not particularly limited, known methods for anaerobic culture of lactic acid bacteria conventionally exist, such as stationary culture in the presence of an oxygen concentration regulator (such as AnaeroPack Kenki, manufactured by Mitsubishi Gas Chemical Company). In the present invention, it is also acceptable to re-inoculate the culture solution after the aforementioned culture into the first skimmed milk powder medium, and perform the first culture multiple times (for example, up to 2 times).

### [Culture (Second Culture)]

In conditions (a) and (b), culture is performed using lactic acid bacteria or the culture solution after the first culture (that is, the first 10% skimmed milk powder medium comprising activated lactic acid bacteria) (hereinafter sometimes referred to as "second culture"). In the second culture, culture is performed at 43°C in a 10% skimmed milk powder medium (hereinafter, the medium for the second culture is sometimes referred to as "second 10% skimmed milk powder medium"). In this case, the amount of lactic acid bacteria subjected to the second culture is preferably an amount that results in a viable bacterial count of 2 × 10⁶ to 5 × 10⁷ cfu/g, more preferably 4 × 10⁶ to 4 × 10⁷ cfu/g, and more preferably 1 × 10⁷ to 2 × 10⁷ cfu/g, in the second 10% skimmed milk powder medium. The culture method in the second culture is not particularly limited and can include fermentation methods for fermented milk using conventional lactic acid bacteria, such as stationary culture, as appropriate.

In the second culture, when fermentation occurs due to the metabolism of lactic acid bacteria producing lactic acid from lactose comprised in the second 10% skimmed milk powder medium, the pH of the culture solution decreases due to the produced lactic acid. In the second culture according to conditions (a) and (b), the completion of this fermentation is determined by the pH of the culture solution (that is, the pH of the second 10% skimmed milk powder medium comprising lactic acid bacteria) becoming 4.7 or lower.

Conditions (a1) and (b3) require that the time until completion of the fermentation (that is, when pH reaches 4.7) is 7 hours or less from the start of the second culture, particularly preferably 6 hours or less, and further preferably 5 hours or less. For lactic acid bacteria that exceed the upper limit in the time until completion of the fermentation, it is not preferable for production efficiency because it takes time to produce when used in the production of fermented milk, and the viable bacterial count in the fermented milk decreases upon low-temperature storage. While there is no particular limitation on the lower limit of the time until completion of the fermentation, for instance, it is preferable to be 1 hour or more, and more preferable to be 2 hours or more.

When the second culture continues for 20 hours or more, typically, the pH of the culture solution further decreases due to the continued production of lactic acid and the following increase in this produced lactic acid. In contrast, under conditions (a2) and (b4), it is necessary for the pH of the culture solution 20 hours after the start of the second culture to be 4.1 or higher, and it is particularly preferable for the pH of the culture solution 24 hours after the start of culture to be 4.1 or higher. Moreover, there is no particular limitation on the upper limit of the time from the start of the second culture to maintain a pH of 4.1 or higher, but for example, it is preferable to be 30 hours or less. The present inventors have found that lactic acid bacteria that can maintain a pH of 4.1 or higher even after long-term culture at 43°C can also suppress the decrease in pH during low-temperature (such as 10°C) storage of the resulting fermented milk. Note that even lactic acid bacteria with low fermentation capability may maintain a pH of 4.1 or higher after long-term culture at 43°C, but in this case, at the very least, conditions (a1) and (b3), and/or conditions (b1) and (a3) are not satisfied.

### [Storage]

In conditions (a3), (a4), (b1), and (b2), the culture solution after the fermentation is completed (that is, after reaching pH 4.7) is promptly cooled and stored. While the conditions for storage are not particularly limited, methods known as conventional fermented milk storage methods can be mentioned, such as a method involving sealing (preferably airtight sealing) the culture solution in a container and allowing it to stand.

As for the storage temperature according to conditions (a3), (a4), (b1), and (b2), it is necessary for it to be a low temperature, more specifically, it needs to be 10°C. Additionally, for the storage period based on these conditions, it is necessary for each independently to be 10 days or more, particularly preferably 16 days or more, more preferably 20 days or more, and further preferably 35 days or more. In the lactic acid bacterium of the present invention, even when stored for a long period under such low-temperature conditions, the amount of acid production is sufficiently low, and the viable bacterial count is adequately maintained.

Since lactic acid bacteria can typically produce the aforementioned lactic acid even at low temperatures, when the culture solution after completion of fermentation is stored for a long time as mentioned above, even under low-temperature conditions, the pH of the culture solution further decreases with an increase in the amount of the lactic acid produced. In contrast, in conditions (a4) and (b2), it is necessary for the pH of the culture solution (that is, the pH of the second 10% skimmed milk powder medium comprising lactic acid bacteria) after being stored for 10 days at 10°C to be 4.2 or higher (preferably 4.3 or higher, more preferably 4.4 or higher), and it is particularly preferable for the pH of the culture solution after being stored for 16 days at 10°C to be 4.2 or higher (preferably 4.3 or higher, more preferably 4.4 or higher), and it is further preferable for the pH of the culture solution after being stored for 35 days at 10°C to be 4.2 or higher (preferably 4.3 or higher, more preferably 4.4 or higher).

Among the conventional lactic acid bacteria, there are lactic acid bacteria known for their sensitivity to low temperatures, in which case even when stored at low temperatures for extended periods, the pH of the culture solution may not decrease. However, the present inventors have newly discovered that conventionally known low-temperature-sensitive lactic acid bacteria, such as those described in PTL 2, may face the problem of a long time required to complete fermentation or a reduced viable bacterial count due to low-temperature storage. Regarding this, the present inventors speculate that in conventionally known low-temperature-sensitive lactic acid bacteria, such as the bacteria described in PTL 3, if the pH decreases, apoptosis occurs where H⁺ is not discharged outside the bacterial body, leading to a decrease in the pH in the bacterial body and inhibiting growth. In contrast, the present inventors have found lactic acid bacteria that can suppress the decrease in pH during low-temperature storage and can sufficiently maintain viable bacterial count during such low-temperature storage, that is, lactic acid bacteria that satisfy condition (a3) or (b1). In conditions (a3) and (b1), it is necessary for the viable bacterial count in the culture solution after being stored for 10 days at 10°C to be 1 × 10⁸ cfu/g or higher, it is particularly preferable for the viable bacterial count in the culture solution after being stored for 16 days at 10°C to be 1 × 10⁸ cfu/g or higher, it is more preferable for the viable bacterial count in the culture solution after being stored for 20 days at 10°C to be 1 × 10⁸ cfu/g or higher, and it is further preferable for the viable bacterial count in the culture solution after being stored for 35 days at 10°C to be 1 × 10⁸ cfu/g or higher. Note that the conventionally known low-temperature-sensitive lactic acid bacteria at least do not satisfy conditions (a1) and (b3), and/or conditions (b1) and (a3).

The lactic acid bacterium of the present invention, being S. thermophilus, which satisfy the conditions (a) and/or (b) may be a single strain or a combination of two or more strains. Specifically, more specific examples of lactic acid bacterium of the present invention include Streptococcus thermophilus OLS4801 (hereinafter sometimes referred to as "OLS4801 strain"), specified by accession number NITE BP-03504, Streptococcus thermophilus OLS4802 (hereinafter sometimes to as "OLS4802 strain"), specified by accession number NITE BP-03505, Streptococcus thermophilus OLS4803 (hereinafter sometimes to as "OLS4803 strain"), specified by accession number NITE BP-03506, Streptococcus thermophilus OLS4823 (hereinafter sometimes to as "OLS4823 strain"), specified by accession number NITE BP-03507, and Streptococcus thermophilus OLS4824 (hereinafter sometimes to as "OLS4824 strain"), specified by accession number NITE BP-03508. All of these exemplified lactic acid bacteria are S. thermophilus, possess the prtS gene, and fulfill the conditions (a) and (b).

Each strain has been deposited, that is, OL4801 strain: (1) identification label: Streptococcus thermophilus OL4801, (2) accession number: NITE BP-03504, (3) accession date: August 4, 2021, OL4802 strain: (1) identification label: Streptococcus thermophilus OL4802, (2) accession number: NITE BP-03505, (3) accession date: August 4, 2021, OL4803 strain: (1) identification label: Streptococcus thermophilus OL4803, (2) accession number: NITE BP-03506, (3) accession date: August 4, 2021, OL4823 strain: (1) identification label: Streptococcus thermophilus OL4823, (2) accession number: NITE BP-03507, (3) accession date: August 4, 2021, and OL4824 strain: (1) identification label: Streptococcus thermophilus OL4824, (2) accession number: NITE BP-03508, (3) accession date: August 4, 2021, at (4) depositary institution: National Institute of Technology and Evaluation NITE Patent Microorganisms Depositary (postal code 292-0818, Room 122, 2-5-8 Kazusa Kamatari, Kisarazu City, Chiba Prefecture). Note that the S. thermophilus identified by these accession numbers may be a subcultured strain of the same strain, or may be an artificial mutant strain, a natural mutant strain, a genetically modified strain, a derivative strain, or the like of the same strain or a subcultured strain thereof, as long as the effects of the present invention are not impaired.

### <Method for Screening Lactic Acid Bacterium>

In lactic acid bacterium that satisfy the above condition (a) and/or (b), during low-temperature storage, the amount of acid production is low and the viable bacterial count is not reduced even by the low-temperature storage. Therefore, using these conditions as indicators, it becomes possible to screen for suitable lactic acid bacterium for producing fermented milk in which decrease in pH during low-temperature storage is suppressed and the viable bacterial count of lactic acid bacteria is sufficiently maintained. Accordingly, the present invention also provides a method for screening lactic acid bacterium, being S. thermophilus, including a selection step of selecting lactic acid bacterium with low acid production using an indicator that at least one selected from the group consisting of conditions (a) and (b) is satisfied. In the present invention, "lactic acid bacterium (bacteria) with low acid production" refers to lactic acid bacterium that, when used, can suppress the decrease in pH during low-temperature storage and maintain viable bacterial counts in the resulting fermented milk.

In the aforementioned selection step, lactic acid bacterium of the present invention undergo a second culture, and culture (and if necessary, first culture) and storage are carried out under the respective conditions mentioned in Storage, and lactic acid bacterium that satisfy condition (a) and/or (b) are determined and selected as the lactic acid bacterium with low acid production. The conditions (a) and (b) are as described in the lactic acid bacterium of the present invention, including their preferable embodiments. Among these, in the selection step according to the present invention, selecting lactic acid bacteria, being S. thermophilus, that satisfy condition (a) as the lactic acid bacterium with low acid production is preferable, and it is preferable to further satisfy condition (a3) and/or condition (a4) as condition (a), and more preferably further satisfy condition (b).

### <Lactic Acid Bacterium Composition>

The term "lactic acid bacterium composition" refers to a composition comprising lactic acid bacterium, without particular limitations, and as the lactic acid bacterium composition of the present invention, it suffices to comprise at least the lactic acid bacterium of the present invention (including lactic acid bacterium selected by the method for screening lactic acid bacterium of the present invention), and may be composed solely of the lactic acid bacterium (bacteria) of the present invention (including a combination of two or more types of lactic acid bacteria of the present invention). The lactic acid bacterium composition of the present invention encompasses, for example, the following lactic acid bacterium starter and fermented milk. As the lactic acid bacterium composition of the present invention, it is also acceptable to further comprise additional components other than the lactic acid bacterium of the present invention, and these additional components are not particularly limited and may include solvents such as water; cultures such as the supernatant of the culture solution after the lactic acid bacteria have finished culturing, and culture medium components; concentrated, diluted, dried, frozen forms of the culture, and the like, and these can be singular or in combinations of two or more.

### (Lactic Acid Bacterium Starter)

The lactic acid bacterium starter of the present invention, which comprises at least the lactic acid bacterium of the present invention, can be suitably used for the method for producing fermented milk of the present invention described later. As such lactic acid bacterium starter of the present invention, it may be composed solely of the lactic acid bacterium (bacteria) of the present invention, or it may further comprise the aforementioned additional components, and additional lactic acid bacteria or yeast.

Regarding additional lactic acid bacteria and yeast, known lactic acid bacteria and yeast that have conventionally been included in fermented milk are mentioned. Examples of additional lactic acid bacteria include, in addition to S. thermophilus other than the lactic acid bacterium of the present invention, lactic acid bacteria of the genus Lactobacillus (such as Lactobacillus delbrueckii (especially Lactobacillus delbrueckii subsp. bulgaricus (Bulgarian bacterium), Lactobacillus delbrueckii subsp. delbrueckii, Lactobacillus delbrueckii subsp. lactis, Lactobacillus delbrueckii subsp. indicus, Lactobacillus delbrueckii subsp. sunkii, Lactobacillus delbrueckii subsp. jakobsenii, and the like), Lactobacillus helveticus, Lactobacillus acidophilus, Lactobacillus johnsonii, Lactobacillus gasseri, Lactobacillus paragasseri, Lactobacillus amylovorus, and Lactobacillus crispatus), lactic acid bacteria of the genus Lacticaseibacillus, lactic acid bacteria of the genus Lactiplantibacillus, lactic acid bacteria of the genus Liquorilactobacillus, lactic acid bacteria of the genus Latilactobacillus, lactic acid bacteria of the genus Ligilactobacillus, lactic acid bacteria of the genus Limosilactobacillus, lactic acid bacteria of the genus Lentilactobacillus, lactic acid bacteria of the genus Levilactobacillus, lactic acid bacteria of the genus Pediococcus, lactic acid bacteria of the genus Leuconostoc, and lactic acid bacteria of the genus Lactococcus, and these can be singular or in combinations of two or more. Among these, as a lactic acid bacterium starter of the present invention, it is preferable to comprise lactic acid bacteria of the genus Lactobacillus, and more preferably, to comprise lactic acid bacteria belonging to Lactobacillus delbrueckii subsp. bulgaricus.

The lactic acid bacterium starter of the present invention may be in a liquid state or in a solid state such as a frozen state or a dry powder, and may further comprise additional components. Examples of the further additional components that can be comprised in the lactic acid bacterium starter include fermentation promoting substances (such as formic acid and nucleic acids); protective agents (saccharides); and medium components (such as milk and whey), and may be one of these or a combination of two or more thereof.

The content of the lactic acid bacteria of the present invention in the lactic acid bacterium starter of the present invention is not particularly limited, but preferably 0.01 to 100% by mass, and more preferably 0.1 to 90% by mass. Also, in terms of the viable bacterial count, it is preferable to be 1 × 10⁷ cfu/g or more, and more preferably 1 × 10⁷ to 1 × 10¹¹ cfu/g. Furthermore, in the case of further comprising additional lactic acid bacteria (preferably lactic acid bacteria of the genus Lactobacillus), the ratio of the content of the lactic acid bacteria of the present invention in the lactic acid bacterium starter and the content of the additional lactic acid bacteria is preferably 1:0.1 to 1:100, more preferably 1:1 to 1:10, in a bacterial count ratio (in terms of viable bacterial count, hereinafter the same).

The lactic acid bacterium starter of the present invention may, for example, be combined with a composition comprising the additional lactic acid bacteria (second lactic acid bacteria composition) to form a lactic acid bacterium starter kit including the lactic acid bacterium starter of the present invention and the second lactic acid bacteria composition. Additionally, it may be, for example, in the form of a lactic acid bacterium starter kit including additives for producing fermented milk (such as fermentation promoting substances and protective agents described above), containers, and usage instructions for the lactic acid bacterium starter.

### (Fermented Milk)

The fermented milk of the present invention comprises at least the lactic acid bacterium of the present invention, and for example, can be obtained by the method for producing fermented milk of the present invention described later. In the fermented milk of the present invention, the decrease in pH during low-temperature storage is suppressed, and the viable bacterial count comprised even by the low-temperature storage is sufficiently maintained.

The fermented milk of the present invention is not particularly limited, and examples thereof include fermented milk that satisfies the standards for "fermented milk" according to the Ministerial Ordinance on Milk and Milk Products (more specifically, the content of milk solids-not-fat is 8.0% or more, and the lactic acid bacteria count or yeast count (preferably the lactic acid bacteria count (more preferably the count of the lactic acid bacteria of the present invention), hereinafter the same) is 10 million/mL or more). In addition, the fermented milk of the present invention also includes those that satisfy the standards for "milk products and fermented milk drinks" (more specifically, the content of milk solids-not-fat is 3.0% or more, and the lactic acid bacteria count or yeast count is 10 million/mL or more); those that satisfy the standards for "fermented milk drinks" (more specifically, the content of milk solids-not-fat is 3.0% or more, and the lactic acid bacteria count or yeast count is 1 million/mL or more), according to the above Ministerial Ordinance on Milk and Milk Products. Note that the milk solids-not-fat refers to the remaining components (mainly such as proteins, lactose, and minerals) obtained by subtracting the fat content from the total milk solids, and the lactic acid bacteria and yeast count are viable bacterial counts measured using the test method stipulated in the Ministerial Ordinance on Milk and Milk Products and the method using the BCP added plate count agar medium, and in the case of fermented milk that has undergone partial sterilization, they are in terms of the viable bacterial counts measured by the aforementioned test method before the partial sterilization.

The fermented milk of the present invention may be a fermented product after the fermentation step of the method for producing fermented milk described later or may be obtained by partially sterilizing the fermented product (such as pulverization or heat treatment), or may be obtained by, for example, concentrating, diluting, drying, or freezing them. Note that in the present invention, when the fermented milk is partially sterilized, the lactic acid bacteria count in the fermented milk is in terms of viable bacterial count. The lactic acid bacteria comprised in the fermented milk of the present invention include not only viable bacteria but also dead bacteria, as well as disrupted products and heat-treated products of lactic acid bacteria, and concentrates, dilutions, dried products, and frozen products thereof. The fermented milk of the present invention preferably comprises at least viable bacteria of lactic acid bacteria, and more preferably comprises the lactic acid bacteria of the present invention as viable bacteria.

The fermented milk of the present invention comprises components derived from the lactic acid bacterium (bacteria) of the present invention and the raw material milk (preferably formula milk) described later. Moreover, within the scope not inhibiting the effects of the present invention, it may further comprise additional components listed in Lactic Acid Bacterium Starter mentioned above, as well as additional lactic acid bacteria and yeast. In addition, it may comprise various components that can be comprised in food and drink. Further components that can be comprised in fermented milk are not particularly limited, and examples thereof include saccharides, sugar alcohols, minerals, vitamins, proteins, peptides, amino acids, organic acids, pH adjusters, starches and modified starches, dietary fibers, fruits and vegetables and processed products thereof, animal and plant crude drug extracts, naturally-derived polymers (such as collagen, hyaluronic acid, and chondroitin), oils and/or fats, thickeners, emulsifiers, solvents, surfactants, gelling agents, stabilizers, buffers, suspending agents, thickening agents, excipients, disintegrators, binders, flow agents, preservatives, colorants, fragrances, corrigents, and sweeteners, and may be one of these or a combination of two or more thereof.

Such fermented milk is preferably yoghurt, cheese, fermented cream, fermented butter, and the like, and particularly preferably yogurt. Specific examples of the yogurt include set type yogurt (solid fermented milk) such as plain yogurt, soft-type yogurt (pasty fermented milk), and drink type yogurt (liquid fermented milk), and frozen yogurt using these ingredients may also be used. In addition, the fermented milk of the present invention can also be used as an ingredient for fermented food such as cheese, fermented cream, fermented butter, and kefir. Furthermore, the fermented milk of the present invention can also be used as a lactic acid bacterium starter in the method for producing fermented milk, provided that it has not been subjected to partial sterilization.

### <Method for Producing Fermented Milk>

The method for producing fermented milk of the present invention includes a fermentation step of adding the lactic acid bacterium of the present invention (including lactic acid bacterium selected by the method for screening lactic acid bacterium of the present invention) or the lactic acid bacterium composition of the present invention to a formula milk comprising raw material milk, followed by fermentation to thereby obtain fermented milk. Note that in the case of using the lactic acid bacterium selected by the method for screening lactic acid bacterium of the present invention, the method for producing fermented milk of the present invention may include the selection step, and the selection step may be only the initial step. According to the production method of the present invention, by using the lactic acid bacterium of the present invention to ferment the raw material milk, it becomes possible to produce fermented milk in which the decrease in pH during low-temperature storage is suppressed, and the viable bacterial count comprised even by the low-temperature storage is sufficiently maintained. Furthermore, the production method of the present invention also makes it possible to significantly shorten the fermentation time until completion.

### (Formula Milk)

The formula milk according to the present invention comprises raw material milk. The raw material milk preferably comprises lactose, and examples thereof include raw milk (such as milk of cow, water buffalo, sheep, goat, and the like), partially sterilized milk, whole milk, skim milk, whey, and processed products thereof (such as whole milk powder, full-fat concentrated milk, skimmed milk powder, skimmed concentrated milk, condensed milk, whey powder, buttermilk, butter, cream, cheese, whey protein concentrate (WPC), whey protein isolate (WPI), α-lactalbumin (α-La), and β-lactoglobulin (β-Lg)), and may be one of these or a mixture of two or more thereof. The formula milk according to the present invention may be composed only of the above raw material milk, or may be an aqueous solution, diluted solution, or concentrated solution of the above raw material milk, or may further comprise additional components in addition to the above raw material milk, if necessary. Examples of the additional components include water; foods, food ingredients, or food additives such as soymilk, saccharides such as sugar, sweeteners, fragrances, fruit juices, fruit pulps, vitamins, minerals, oils and/or fats, ceramides, collagen, milk phospholipids, and polyphenols; stabilizers, thickeners, and gelling agents such as pectin, soy polysaccharides, CMC (carboxymethylcellulose), agar, gelatin, carrageenan, and gums, and may be one of these or a mixture of two or more thereof. The formula milk can be prepared by mixing the above components, while optionally with heating and/or optionally with homogenizing. In addition, as the formula milk, heat total sterilized or partial sterilized one can also be used.

### (Fermentation)

In the fermentation step of adding the lactic acid bacterium of the present invention or the lactic acid bacterium composition of the present invention to the aforementioned formula milk, followed by fermentation, conventionally known methods can be appropriately employed without particular limitations. Moreover, the amount of the lactic acid bacterium of the present invention or the lactic acid bacterium composition of the present invention to be added can be appropriately set according to the amount of lactic acid bacterium starter used in the conventionally known method for producing fermented milk, but is, for example, preferably 1 × 10⁷ to 5 × 10⁹ cfu/g, more preferably 1 × 10⁸ to 2 × 10⁹ cfu/g, in a viable bacterial count of the lactic acid bacteria of the present invention, based on the mass of the formula milk.

To the formula milk, in addition to the lactic acid bacterium of the present invention or the lactic acid bacterium composition of the present invention, the additional lactic acid bacteria and yeast may be added. Among these, for the method for producing fermented milk of the present invention, it is preferable to further add lactic acid bacteria of the genus Lactobacillus, and more preferably to further add lactic acid bacteria belonging to Lactobacillus delbrueckii subsp. bulgaricus. Furthermore, in the case of further adding additional lactic acid bacteria (preferably lactic acid bacteria of the genus Lactobacillus), the ratio of the amount of the lactic acid bacteria of the present invention and the amount of the additional lactic acid bacteria is preferably 1:0.1 to 1:100, more preferably 1:1 to 1:10, in a bacterial count ratio (in terms of viable bacterial count, hereinafter the same).

The fermentation conditions are not particularly limited, and can be appropriately selected depending on the growth conditions of the lactic acid bacterium of the present invention to be added, the amount of the formula milk, and the like. For example, under aerobic or anaerobic conditions at a temperature of 35 to 45°C and more preferably at a temperature of 38 to 43°C, the mixture is allowed to stand or stirred (preferably stand) for usually 2 to 24 hours, more preferably 3 to 8 hours, and further preferably 4 to 6 hours until the pH of the formula milk added with the lactic acid bacterium of the present invention or the lactic acid bacterium composition of the present invention is 4.7 or less, more preferably 4.0 to 4.5. According to the present invention, it is possible to sufficiently shorten the time required for fermentation. In addition, as the anaerobic conditions, for example, fermentation under nitrogen aeration conditions can be employed.

The fermented milk of the present invention can be obtained by the above fermentation. The fermented product after the fermentation step can be used as the fermented milk of the present invention as it is or by concentrating, diluting, drying, freezing, or the like as necessary. Also, the fermented milk of the present invention may be obtained by disrupting or heat-treating the lactic acid bacteria in the fermented product, or by concentrating, diluting, drying, freezing, or the like as necessary. Therefore, the method for producing fermented milk of the present invention may further include these steps (such as concentration step, dilution step, drying step, freezing step, disrupting step, and heat treatment step) .

### [Examples]

Hereinafter, the present invention will be described in more detail based on the Examples and Comparative Examples, but the present invention is not limited to the following Examples.

### (Test Example 1)

(1) The following three strains of lactic acid bacteria were used:
   P2101201 strain (Comparative Example 1): S. thermophilus possessing the prtS gene. The bacterial strain is preserved by the Meiji Innovation Center of Meiji Co., Ltd. (postal code 192-0919, 1-29-1 Nanakuni, Hachioji City, Tokyo, Japan);
   OLS4801 strain (Example 1): S. thermophilus specified by accession number NITE BP-03504. It possesses the prtS gene; and
   OLS4802 strain (Example 2): S. thermophilus specified by accession number NITE BP-03505. It possesses the prtS gene.
   In the test examples described in the present specification, the presence or absence of the prtS gene in each lactic acid bacterial strain was confirmed by the following method. Specifically, first, the prtS gene sequences of 5 strains of S. thermophilus with known genome sequences were obtained from the NCBI database, and the highly conserved sequences were used to prepare primers (Fprimer: SEQ ID NO: 1 and Rprimer: SEQ ID NO: 2). In addition, InstaGene Matrix (manufactured by BioRad) was used to extract genomic DNA from the M17 culture solution of each bacterial strain. 0.5 µL of extracted genomic DNA (template), 1 µL each of the prepared primers (5 µM), 0.1 µL of Phusion high fidelity DNA polymerase, 2 µL of 5 × HF buffer, 0.8 µL of 2.5 mM dNTP, and 4.6 µL of ultrapure water were mixed (total 10 µL), and PCR was performed under the following conditions: at 98°C for 30 seconds; 30 cycles of 98°C for 5 seconds, 63°C for 20 seconds, and 72°C for 20 seconds; and 7 2° C for 5 minutes; and allowed to stand at 4°C. The resulting PCR products were subjected to agarose gel electrophoresis, and bacterial strains confirmed to have a band at 684 bp were determined to have the prtS gene, and bacterial strains not confirmed to have the band were determined not to have the prtS gene.
(2) In a 10% skimmed milk powder medium that had been subjected to total sterilization at 121°C for 7 minutes (10 parts by mass of skimmed milk powder and 90 parts by mass of distilled water (10% by mass of skimmed milk powder), pH: 6 to 7, hereinafter the same), the three strains of lactic acid bacteria were individually subjected to stationary culture overnight (16 hours, hereinafter the same) at 37°C under anaerobic conditions (using an oxygen concentration regulator (AnaeroPack Kenki, manufactured by Mitsubishi Gas Chemical Company), hereinafter the same) for activation. Note that the bacterial count in the culture solution after activation was 5 × 10⁸ to 1 × 10⁹ cfu/g. Subsequently, a 10% skimmed milk powder medium that had been subjected to partial sterilization at 75°C for 15 minutes was inoculated such that the mass of the culture solution was 2% by mass (viable bacterial count of lactic acid bacteria: 1 × 10⁷ to 2 × 10⁷ cfu/g), and subjected to stationary culture (fermentation) at 43°C, and the time (fermentation time) from the inoculation until the pH of the culture solution decreased to 4.7 was measured. Furthermore, the culture was terminated when the pH reached 4.7, and immediately it was cooled down to 10°C, sealed tightly to prevent the entry of air, and allowed to stand for 10 days, 17 days, or 35 days to measure each pH (10°C pH) after storage. Separately from the stored samples, following the inoculation, culture (fermentation) was conducted at 43°C, and the pH of the culture solution (43°C pH) was measured after continuing the culture for up to 24 hours. The results are shown in the following Table 1.

**[Table 1]**

| Lactic Acid Bacteria | Fermentation Time (Min) | 43°C pH (24 hr) | 10°C pH | | |
|---|---|---|---|---|---|
| | | | 10 Days | 17 Days | 35 Days |
| P2101201 | 240 | 4.00 | 4.15 | 4.11 | 4.11 |
| OLS4801 | 245 | 4.16 | 4.44 | 4.42 | 4.41 |
| OLS4802 | 235 | 4.12 | 4.45 | 4.42 | 4.42 |

As shown in Table 1, fermentation was completed within 5 hours for each lactic acid bacterium (reaching a pH of 4.7). On the other hand, regarding the time course of pH during storage at 10°C, compared to the OL4801 strain and OL4802 strain where the pH did not drop below 4.4 even after 35 days of storage, the pH of the P2101201 strain decreased, reaching as low as 4.11 after 17 days of storage. Here, after 24 hours of culture at 43°C, the pH for both OL4801 strain and OL4802 strain remained at 4.1 or higher, whereas for the P2101201 strain, the pH after 24 hours of culture at 43°C dropped to 4.00, so that the lactic acid bacteria that exhibit a pH of 4.1 or higher after prolonged culture at 43°C (such as 24 hours) suggest sufficient suppression of acid production at low temperatures (such as 10°C).

### (Test Example 2)

In order to confirm the relationship between pH after long-term culture at 43°C and acid production during low-temperature storage, a total of 8 strains of lactic acid bacteria (each is S. thermophilus possessing the prtS gene) including the following 2 strains of lactic acid bacteria:
P2101201 strain (Comparative Example 1); and
OLS4803 strain (Example 3): S. thermophilus specified by accession number NITE BP-03506, possessing the prtS gene
were individually subjected to stationary culture overnight at 37°C under anaerobic conditions for activation, in a 10% skimmed milk powder medium that had been subjected to total sterilization at 121°C for 7 minutes. Subsequently, a 10% skimmed milk powder medium that had been subjected to partial sterilization at 75°C for 15 minutes was inoculated such that the mass of the culture solution was 2% by mass (viable bacterial count of lactic acid bacteria: 1 × 10⁷ to 2 × 10⁷ cfu/g), and subjected to stationary culture (fermentation) at 43°C, and the time (fermentation time) until the pH of the culture solution decreased to 4.7 was measured. Furthermore, the culture was terminated when the pH reached 4.7, and immediately it was cooled down to 10°C, sealed tightly to prevent the entry of air, and allowed to stand for 10 days, 20 days, or 35 days to measure each pH (10°C pH) after storage. Separately from the stored samples, following the inoculation, culture (fermentation) was conducted at 43°C, and the pH of the culture solution (43°C pH) was measured after continuing the culture for up to 20 or 24 hours. Among the lactic acid bacteria tested, the results of the above two strains are shown in the following Table 2.

**[Table 2]**

| Lactic Acid Bacteria | Fermentation Time (Min) | 43°C pH | | 10°C pH | | |
|---|---|---|---|---|---|---|
| | | 20 hr | 24 hr | 10 Days | 20 Days | 35 Days |
| P2101201 | 205 | 4.03 | 4.00 | 4.18 | 3.94 | 3.93 |
| OLS4803 | 235 | 4.25 | 4.25 | 4.57 | 4.52 | 4.51 |

As shown in Table 2, fermentation was also completed within 5 hours for the OL4803 strain (pH reached 4.7). Regarding the time course of pH during storage at 10°C as well, even after 35 days of storage, the pH did not drop below 4.5 for the OL4803 strain. Furthermore, in the case of the OL4803 strain as well, the pH after 20 and 24 hours of culture at 43°C was 4.1 or higher. Additionally, among the total of 8 strains of lactic acid bacteria, one strain of the lactic acid bacteria other than the OL4803 strain completed fermentation within 5 hours, and even after 35 days of storage at 10°C, the pH remained at 4.2 or higher, and the pH after 20 and 24 hours of culture at 43°C was 4.1 or higher. In contrast, for the P2101201 strain and the remaining 5 strains of lactic acid bacteria, fermentation was completed within 5 hours in all cases, but after extended storage at 10°C, the pH dropped below 4.2, and after 20 and 24 hours of culture at 43°C, the pH also dropped below 4.1. From these results, it can be concluded that lactic acid bacteria with a pH of 4.1 or higher after at least 20 hours of culture at 43°C exhibit suppressed acid production at low temperatures (such as 10°C).

### (Test Example 3)

The time course in viable bacterial count during storage at 10°C was examined for OLS4802 strain (Example 2), OLS4803 strain (Example 3), and P2101201 strain (Comparative Example 1). Specifically, each strain was cultured (fermented) similarly to Test Examples 1 and 2, and culturing was terminated when the pH reached 4.7, and at the time of culturing termination (Day 0) and immediately after culturing termination, the cultures were cooled to 10°C, sealed without air ingress, and allowed to stand for 4 days, 11 days, 20 days, or 35 days for storage. After each storage period, the cultured solution was diluted to prepare a suspension, which was spread onto a BCP added plate count agar medium (manufactured by Eiken Chemical Co., Ltd.) and cultured. Colony counts were then enumerated to measure the viable bacterial count (cfu/g) in each culture solution (g). The results are shown in Fig. 1.

As shown in Fig. 1, the OLS4803 strain showed a slight decrease in viable bacterial count compared to other strains after 35 days of storage, but for all lactic acid bacteria, the viable bacterial count after 35 days of storage never dropped below 1 × 10⁸ cfu/g (1.E + 08 cfu/g, hereinafter the same). This confirms its suitability for producing fermented milk while ensuring the standards of fermented milk as per the "Ministerial Ordinance on Milk and Milk Products Concerning Compositional Standards, etc. (Ministerial Ordinance on Milk and Milk Products)." Furthermore, conventionally, the mechanism of acid production inhibition in lactic acid bacteria at low temperatures was known to involve apoptosis due to factors such as a decrease in ATPase activity, as described in PTL 3. However, the lactic acid bacteria of the present invention (such as OLS4802 strain and OLS4803 strain) maintain a sufficient viable bacterial count even at such low temperatures. This suggests that they have acquired a different mechanism for inhibiting acid production at low temperatures.

### (Test Example 4)

First, a total of 7 strains of lactic acid bacteria (each is S. thermophilus possessing the prtS gene) including the following 3 strains of lactic acid bacteria:
OLS4496 strain (Comparative Example 2): S. thermophilus specified by accession number NITE BP-02875, with (1) identification label: Streptococcus thermophilus OLS4496, (2) accession number: NITE BP-02875, and (3) accession date: February 5, 2019, deposited at (4) depositary institution: National Institute of Technology and Evaluation NITE Patent Microorganisms Depositary (postal code 292-0818, Room 122, 2-5-8 Kazusa Kamatari, Kisarazu City, Chiba Prefecture), possessing the prtS gene;
OLS4823 strain (Example 4): S. thermophilus specified by accession number NITE BP-03507, possessing the prtS gene; and
OLS4824 strain (Example 5): S. thermophilus specified by accession number NITE BP-03508, possessing the prtS gene.
were individually subjected to stationary culture overnight at 37°C under anaerobic conditions for activation, in a 10% skimmed milk powder medium that had been subjected to total sterilization at 121°C for 7 minutes. Subsequently, a 10% skimmed milk powder that had been subjected to partial sterilization at 75°C for 15 minutes was inoculated such that the mass of the culture solution was 2% by mass (viable bacterial count of lactic acid bacteria: 1 × 10⁷ to 2 × 10⁷ cfu/g), and subjected to culture (fermentation) at 43°C, and the time (fermentation time) until the pH of the culture solution decreased to 4.7 was measured. Furthermore, the culture was terminated when the pH reached 4.7, and immediately it was cooled down to 10°C, sealed tightly to prevent the entry of air, and allowed to stand for 1 day, 12 days, 24 days, or 40 days to measure each pH (10°C pH) after storage. Separately from the stored samples, following the inoculation, stationary culture (fermentation) was conducted at 43°C, and the pH of the culture solution (43°C pH) was measured after continuing the culture for up to 24 hours. Among the lactic acid bacteria tested, the results of the above three strains are shown in the following Table 3.

**[Table 3]**

| Lactic Acid Bacteria | Fermentation Time (Min) | 43°C pH (24 hr) | 10°C pH | | | |
|---|---|---|---|---|---|---|
| | | | 1 Day | 12 Days | 24 Days | 40 Days |
| OLS4496 | 265 | 4.01 | 4.47 | 4.18 | 4.18 | 4.12 |
| OLS4823 | 315 | 4.43 | 4.63 | 4.56 | 4.55 | 4.52 |
| OLS4824 | 275 | 4.16 | 4.55 | 4.52 | 4.48 | 4.49 |

As shown in Table 3, fermentation was completed within 7 hours for both the OLS4823 strain and OLS4824 strain (pH reached 4.7), and even after 40 days of storage, the pH did not drop below 4.4. Furthermore, in the case of both OLS4823 strain and OLS4824 strain as well, the pH after 24 hours of culture at 43°C was 4.1 or higher. Additionally, among the total of 7 strains of lactic acid bacteria, another strain of lactic acid bacteria other than the two mentioned completed fermentation within 7 hours, and even after 40 days of storage at 10°C, the pH was 4.2 or higher, and after 24 hours of culture at 43°C, the pH was 4.1 or higher. In contrast, for the OLS4496 strain and the remaining 3 strains of lactic acid bacteria, fermentation was completed within 7 hours in all cases, but after extended storage at 10°C, the pH dropped below 4.2, and after 24 hours of culture at 43°C, the pH dropped below 4.1. These results further confirm that lactic acid bacteria with a pH of 4.1 or higher after extended culture at 43°C (such as 24 hours) exhibit suppressed acid production at low temperatures (such as 10°C).

### (Test Example 5)

First, the following 6 strains of lactic acid bacteria:
P2101201 strain (Comparative Example 1); OLS4801 strain (Example 1); OLS4802 strain (Example 2); and OLS4803 strain (Example 3);
SBT0144 strain (Comparative Example 3): S. thermophilus specified by accession number FERM P-16638 as described in PTL 2; and
1131 strain (Comparative Example 4): S. thermophilus isolated from Meiji Bulgarian Yogurt LB81 (manufactured by Meiji Co., Ltd.)
were individually subjected to stationary culture overnight at 37°C under anaerobic conditions for activation, in a 10% skimmed milk powder medium that had been subjected to total sterilization at 121°C for 7 minutes. Subsequently, a 10% skimmed milk powder that had been subjected to partial sterilization at 75°C for 15 minutes was inoculated such that the mass of the culture solution was 2% by mass (viable bacterial count of lactic acid bacteria: 1 × 10⁷ to 2 × 10⁷ cfu/g), and subjected to stationary culture (fermentation) at 43°C, and the time (fermentation time) until the pH of the culture solution decreased to 4.7 was measured. Furthermore, the culture was terminated when the pH reached 4.7, and immediately it was cooled down to 10°C, sealed tightly to prevent the entry of air, and allowed to stand for 10 days, 17 days, or 36 days to measure each pH (10°C pH) after storage. In addition, the viable bacterial count (cfu/g) in the culture solution (g) after each storage period was measured in the same manner as in Test Example 3. The results are shown in Table 4 below.

**[Table 4]**

| Lactic Acid Bacteria | Fermentation Time (Min) | 10°C pH | | | 10°C Bacterial Count (cfu/g) | | |
|---|---|---|---|---|---|---|---|
| | | 10 Days | 17 Days | 36 Days | 10 Days | 17 Days | 36 Days |
| P2101201 | 230 | 4.15 | 4.06 | 4.05 | 6.6E+08 | 7.2E+08 | 4.9E+08 |
| OLS4801 | 235 | 4.50 | 4.46 | 4.46 | 8.9E+08 | 9.0E+08 | 5.2E+08 |
| OLS4802 | 250 | 4.48 | 4.44 | 4.44 | 1 . 1E+09 | 8.8E+08 | 4.3E+08 |
| OLS4803 | 260 | 4.62 | 4.56 | 4.56 | 8.7E+08 | 8.0E+08 | 4.5E+08 |
| SBT1044 | 990 | 4.54 | 4.48 | 4.45 | 3.5E+07 | 2.8E+07 | 2.9E+07 |
| 1131 | 970 | 4.52 | 4.48 | 4.50 | 6.0E+07 | 4.5E+07 | 2.5E+07 |

As shown in Table 4, similar to Test Examples 1 to 3, fermentation was completed within 5 hours for all of OL4801 strain, OL4802 strain, and OL4803 strain (reaching pH 4.7), and even after 36 days of storage at 10°C, the pH remained at 4.4 or higher. Furthermore, even after 36 days of storage, the viable bacterial count was maintained at or above 1 × 10⁸ cfu/g. On the other hand, for P2101201 strain, fermentation was completed within 5 hours, and although the viable bacterial count did not drop below 1 × 10⁸ cfu/g even after 36 days of storage, the pH dropped below 4.2 after at least 10 days of storage at 10°C. Similarly, for strains 1131 and SBT0144, known as conventional low-temperature-sensitive strains, while the pH remained at 4.4 or higher after 36 days of storage at 10°C, it took 16 hours or longer for fermentation to complete. Additionally, the viable bacterial count of at least 1 × 10⁸ cfu/g could not be maintained after at least 10 days of storage at 10°C for these strains.

### (Test Example 6)

First, the following 4 strains of lactic acid bacteria: OLS4823 strain (Example 4), OLS4824 strain (Example 5), SBT0144 strain (Comparative Example 3), and 1131 strain (Comparative Example 4) were individually subjected to stationary culture overnight at 37°C under anaerobic conditions for activation, in a 10% skimmed milk powder medium that had been subjected to total sterilization at 121°C for 7 minutes. Subsequently, a 10% skimmed milk powder that had been subjected to partial sterilization at 75°C for 15 minutes was inoculated such that the mass of the culture solution was 2% by mass (viable bacterial count of lactic acid bacteria: 1 × 10⁷ to 2 × 10⁷ cfu/g), and subjected to stationary culture (fermentation) at 43°C, and the time (fermentation time) until the pH of the culture solution decreased to 4.7 was measured. Furthermore, the culture was terminated when the pH reached 4.7, and immediately it was cooled down to 10°C, sealed tightly to prevent the entry of air, and allowed to stand for 10 days, 17 days, or 36 days to measure each pH (10°C pH) after storage. In addition, the viable bacterial count (cfu/g) in the culture solution (g) after each storage period was measured in the same manner as in Test Example 3. The results are shown in Table 5 below.

**[Table 5]**

| Lactic Acid Bacteria | Fermentation Time (Min) | 10°C pH | | | 10°C Bacterial Count (cfu/g) | | |
|---|---|---|---|---|---|---|---|
| | | 10 Days | 17 Days | 36 Days | 10 Days | 17 Days | 36 Days |
| OLS4823 | 310 | 4.46 | 4.43 | 4.42 | 5.4E+08 | 5.5E+08 | 3.9E+08 |
| OLS4824 | 325 | 4.52 | 4.49 | 4.49 | 1.0E+09 | 6.5E+08 | 5.9E+08 |
| SBT1044 | 990 | 4.54 | 4.48 | 4.45 | 3.5E+07 | 2.8E+07 | 2.9E+07 |
| 1131 | 970 | 4.52 | 4.48 | 4.50 | 6.0E+07 | 4.5E+07 | 2.5E+07 |

As shown in Table 5, similar to Test Example 4, fermentation was completed within 7 hours for both of OLS4823 strain and OLS4824 strain (reaching pH 4.7), and even after 36 days of storage at 10°C, the pH remained at 4.4 or higher. Furthermore, even after 36 days of storage, the viable bacterial count was maintained at or above 1 × 10⁸ cfu/g. On the other hand, similar to Test Example 5, for strains 1131 and SBT0144, known as conventional low-temperature-sensitive strains, while the pH remained at 4.2 or higher after 36 days of storage at 10°C, it took 16 hours or longer for fermentation to complete. Additionally, the viable bacterial count of at least 1 × 10⁸ cfu/g could not be maintained after at least 10 days of storage at 10°C for these strains.

### [Industrial Applicability]

As described above, the present invention makes it possible to provide lactic acid bacterium which allows for obtaining fermented milk in which the decrease in pH during low-temperature storage is suppressed, and the viable bacterial count of the lactic acid bacteria is sufficiently maintained even by the low-temperature storage, a lactic acid bacterium starter and fermented milk comprising the same, and a method for producing fermented milk, and further a method for screening lactic acid bacterium.

### [Accession Number]

1.
   (1) Identification label: Streptococcus thermophilus OLS4801
   (2) Accession number: NITE BP-03504
   (3) Accession date: August 4, 2021
   (4) Depositary institution: National Institute of Technology and Evaluation NITE Patent Microorganisms Depositary
2.
   (1) Identification label: Streptococcus thermophilus OLS4802
   (2) Accession number: NITE BP-03505
   (3) Accession date: August 4, 2021
   (4) Depositary institution: National Institute of Technology and Evaluation NITE Patent Microorganisms Depositary
3.
   (1) Identification label: Streptococcus thermophilus OLS4803
   (2) Accession number: NITE BP-03506
   (3) Accession date: August 4, 2021
   (4) Depositary institution: National Institute of Technology and Evaluation NITE Patent Microorganisms Depositary
4.
   (1) Identification label: Streptococcus thermophilus OLS4823
   (2) Accession number: NITE BP-03507
   (3) Accession date: August 4, 2021
   (4) Depositary institution: National Institute of Technology and Evaluation NITE Patent Microorganisms Depositary
5.
   (1) Identification label: Streptococcus thermophilus OLS4824
   (2) Accession number: NITE BP-03508
   (3) Accession date: August 4, 2021
   (4) Depositary institution: National Institute of Technology and Evaluation NITE Patent Microorganisms Depositary
6.
   (1) Identification label: Streptococcus thermophilus OLS4496
   (2) Accession number: NITE BP-02875
   (3) Accession date: February 5, 2019
   (4) Depositary institution: National Institute of Technology and Evaluation NITE Patent Microorganisms Depositary

## Claims

1. A lactic acid bacterium belonging to Streptococcus thermophilus, satisfying at least one of the conditions selected from the group consisting of the following (a) and (b):
(a) when cultured at 43°C in a 10% skimmed milk powder medium, (a1) the time from start of the culture until a pH of a culture solution becomes 4.7 or lower is 7 hours or less, and (a2) the pH of the culture solution 20 hours after the start of the culture becomes 4.1 or higher, and
(b) when cultured until the pH of the culture solution becomes 4.7 or less in a 10% skimmed milk powder medium at 43°C, followed by storage at 10°C for 10 days, (b1) a viable bacterial count in the culture solution after the storage becomes 1 × 10⁸ cfu/g or more, and (b2) the pH of the culture solution after the storage becomes 4.2 or higher.

2. The lactic acid bacterium according to claim 1, satisfying the condition (a) and further satisfying the following (a3) or (a4):
(a3) in the culture, after the culture until the pH of the culture solution becomes 4.7 or lower, the viable bacterial count in the culture solution after 10 days of storage at 10°C becomes 1 × 10⁸ cfu/g or higher, or
(a4) in the culture, after the culture until the pH of the culture solution becomes 4.7 or lower, the pH in the culture solution after 10 days of storage at 10°C becomes 4.2 or higher.

3. The lactic acid bacterium according to claim 1, satisfying the condition (b) and further satisfying the following (b3) or (b4):
(b3) the time until the pH of the culture solution becomes 4.7 or lower is 7 hours or less from the start of the culture, or
(b4) the pH of the culture solution after 20 hours from the start of the culture is 4.1 or higher.

4. The lactic acid bacterium according to claim 1, possessing a prtS gene.

5. The lactic acid bacterium according to claim 1, which is at least one selected from the group consisting of Streptococcus thermophilus OLS4801 (accession number: NITE BP-03504), Streptococcus thermophilus OLS4802 (accession number: NITE BP-03505), Streptococcus thermophilus OLS4803 (accession number: NITE BP-03506), Streptococcus thermophilus OLS4823 (accession number: NITE BP-03507), and Streptococcus thermophilus OLS4824 (accession number: NITE BP-03508) .

6. A lactic acid bacterium composition comprising: the lactic acid bacterium according to claim 1.

7. The lactic acid bacterium composition according to claim 6, further comprising: a lactic acid bacterium of the genus Lactobacillus.

8. The lactic acid bacterium composition according to claim 6, which is a lactic acid bacterium starter.

9. The lactic acid bacterium composition according to claim 6, which is fermented milk.

10. A method for producing fermented milk comprising: a fermentation step of adding the lactic acid bacterium according to claim 1 or the lactic acid bacterium composition according to claim 6 to a formula milk comprising raw material milk, followed by fermentation to thereby obtain fermented milk.

11. The method for producing fermented milk according to claim 10, further comprising: adding a lactic acid bacterium of the genus Lactobacillus to the formula milk.

12. A method for screening lactic acid bacterium, comprising: a selection step of selecting lactic acid bacterium with low acid production using an indicator that a lactic acid bacterium belonging to Streptococcus thermophilus satisfies the following condition (a):
(a) when cultured at 43°C in a 10% skimmed milk powder medium, (a1) the time from start of the culture until a pH of a culture solution becomes 4.7 or lower is 7 hours or less, and (a2) the pH of the culture solution 20 hours after the start of the culture becomes 4.1 or higher.

13. A method for producing fermented milk comprising: a fermentation step of adding lactic acid bacterium selected by the method for screening lactic acid bacterium according to claim 12 to a formula milk comprising raw material milk, followed by fermentation to thereby obtain fermented milk.
